# EUROPEAN PATENT APPLICATION

(11) **EP 3 072 880 A1**
(43) Date of publication of application: **28.09.2016**
(21) Application number: 14864543.5
(22) Date of filing: 18.11.2014
(51) Int. Cl.: C07C 209/84, C07C 211/09, C12P 13/00

(54) **1, 5-PENTAMETHYLENE DIAMINE AND METHOD FOR PRODUCING SAME**

(30) Priority: 19.11.2013 JP 2013239245
(71) Applicant: Toray Industries, Inc., Tokyo, 103-8666 (JP); Ajinomoto Co., Inc., Tokyo 104-8315 (JP)
(72) Inventor: MURATA, Yoshiaki, Nagoya-shi Aichi 455-8502 (JP); KURIBAYASHI, Takahiro, Nagoya-shi Aichi 455-8502 (JP); ONISHI, Fumito, Kawasaki-city Kanagawa 210-8681 (JP); HIURA, Takehiro, Kawasaki-city Kanagawa 210-8681 (JP)
(74) Representative: Hoefer & Partner Patentanwälte mbB
(86) International application number: PCT/JP2014/080446
(87) International publication number: WO 2015/076238

(57) **Abstract**

A biomass resource-derived 1,5-pentamethylene diamine that gives a polyamide resin composition having a good color tone and a production method therefor are provided. A 1,5-pentamethylene diamine in which a total of the contents of acetamide, 2,3,4,5-tetrahydropyridine, 2-piperidone, α-amino-ε-caprolactam, lysine, and ornithine that the 1,5-pentamethylene diamine contains as impurities is less than or equal to 150 weight ppm is made.

## Description

### Technical Field

The invention relates to a 1,5-pentamethylene diamine and a production method therefor.

### Background Art

In recent years, due to heightened consciousness toward environmental problems, utilization of biomass resources that are renewable resources as alternatives to petroleum resources has been drawing great attention. In particular, various plastics are utilized in diverse fields, such as fibers, films, motor vehicle component parts, or electrical and electronic component parts. Raw materials for production of these plastics are expected to be replaced by biomass resource-derived ones.

As one of such biomass resource-derived plastics, polyamide resins, such as nylon 56 and nylon 510, for which a biomass resource-derived 1,5-pentamethylene diamine has been used as a monomer component, are cited.

However, in the 1,5-pentamethylene diamine obtained from a biomass resource derivative, small amounts of nitrogen-containing organic compounds represented by biomass resource-derived amino acids or proteins or decomposition products thereof exist as impurities. If a nitrogen-containing organic compound exists, there occurs a problems such as retardation of polymerization, coloration of the polyamide resin obtained, occurrence of appearance defect due to fisheye, decline of heat resistance, decline of melt residence stability, or increase of water absorptiveness.

Therefore, various 1,5-pentamethylene diamines whose nitrogen-containing organic compound content has been reduced and various production methods therefor have been considered. For example, Patent documents 1 and 2 each disclose a 1,5-pentamethylene diamine that is low in the content of the nitrogen-containing organic compounds with three or more functional groups, such as amino acids, contained as impurities and a production method therefor, and disclose that a polyamide resin for which a 1, 5-pentamethylene diamine that is low in the content of nitrogen-containing organic compounds with three or more functional groups has been used as a raw material has less fisheye and provides a film of a polyamide resin excellent in surface appearance. Furthermore, Patent document 3 discloses a 1,5-pentamethylene diamine that is low in the contents of 2,3,4,5-tetrahydropyridine and piperidine contained as impurities and a production method therefore, and discloses a polyamide resin for which a 1,5-pentamethylene diamine that is low in the contents of 2,3,4,5-tetrahydropyridine and piperidine has been used as a raw material and which is excellent in heat resistance, melt residence stability, and low water absorbency.

### Prior Art Documents

### Patent documents

Patent document 1: Japanese Unexamined Patent Publication (Kokai) No. 2008-189918
Patent document 2: Japanese Unexamined Patent Publication (Kokai) No. 2010-275516
Patent document 3: International Publication No. WO 2010/113736

### Summary of the Invention

### Problems to be Solved by the Invention

However, even the polyamide resin for which the 1,5-pentamethylene diamine disclosed in Patent documents 1 and 2 that is low in the content of the nitrogen-containing organic compounds with three or more functional groups, such as amino acids or the like, contained as impurities has been used as a raw material has possibility that the polyamide resin will be colored in the case where the nitrogen-containing organic compounds other than the nitrogen-containing organic compound with three or more functional groups, such as amino acids, have not been sufficiently removed. Furthermore, even in the polyamide resin for which the 1,5-pentamethylene diamine disclosed in Patent document 3 that is low in the contents of 2,3,4,5-tetrahydropyridine and piperidine has been used as a raw material, although 2,3,4,5-tetrahydropyridine and piperidine, which are impurities lower in boiling point than 1,5-pentamethylene diamine, can be removed, impurities higher in boiling point cannot be removed. Therefore, the polyamide resin for which the 1,5-pentamethylene diamine disclosed in Patent document 3 has been used as a raw material has a problem that coloration is exhibited.

Thus, even when a 1,5-pentamethylene diamine disclosed as related-art technologies is used, it is difficult to obtain a polyamide resin that has a good and uniform color tone needed as a commercial product.

Therefore, a task of the invention is to provide a 1,5-pentamethylene diamine that gives a polyamide resin that has a good color tone and a production method therefor.

### Means for Solving the Problems

As a result of implementing vigorous considerations about the foregoing problems, it has been found that using as a raw material a 1, 5-pentamethylene diamine in which the content of specific nitrogen-containing organic compounds contained as impurities is less than or equal to a specific amount will give a polyamide resin having a good color tone, and therefore the invention has been reached.

Namely, the invention is
(1) a 1, 5-pentamethylene diamine characterized in that in which a total content of acetamide, 2,3,4,5-tetrahydropyridine, 2-piperidone, α-amino-ε-caprolactam, lysine, and ornithine contained as impurities is less than or equal to 150 weight ppm.
(2) The 1,5-pentamethylene diamine according to (1) characterized in that a content of the acetamide is less than or equal to 70 weight ppm.
(3) The 1,5-pentamethylene diamine according to one of (1) and (2) mentioned above which is produced from a biomass resource-derived lysine.
(4) A production method for the 1,5-pentamethylene diamine according to any one of (1) to (3), characterized in that the 1, 5-pentamethylene diamine is rectified at a temperature greater than or equal to 40°C and less than or equal to 200°C and a pressure greater than or equal to 0.2 kPa and less than or equal to 1200 kPa and with a theoretical plate number greater than or equal to 5.

### Advantageous Effects of the Invention

According to the invention, a biomass resource-derived 1,5-pentamethylene diamine that gives a polyamide resin having a good color tone can be obtained.

### Description of Preferred Embodiments

As for the 1,5-pentamethylene diamine of the invention, a total content of acetamide, 2,3,4,5-tetrahydropyridine, 2-piperidone, α-amino-ε-caprolactam, lysine, and ornithine contained as impurities (a total of the contents of these compounds) is less than or equal to 150 weight ppm.

### (1,5-Pentamethylene Diamine)

A 1,5-pentamethylene diamine produced from a biomass resource usually contains as impurities nitrogen-containing organic compounds other than 1, 5-pentamethylene diamine. Among those, nitrogen-containing organic compounds, such as acetamide, 2,3,4,5-tetrahydropyridine, 2-piperidone, α-amino-ε-caprolactam, lysine, and ornithine, become a cause of polymerization retardation and coloration in the case of polymerizing a polyamide resin in which 1,5-pentamethylene diamine is used.

Therefore, in order to obtain a polyamide resin that is good in color tone, it is necessary to use a 1, 5-pentamethylene diamine in which the contents of acetamide, 2,3,4,5-tetrahydropyridine, 2-piperidone, α-amino-ε-caprolactam, lysine, and ornithine contained as impurities are small. That is, in the 1,5-pentamethylene diamine of the invention, the total of the contents of acetamide, 2,3,4,5-tetrahydropyridine, 2-piperidone, α-amino-ε-caprolactam, lysine, and ornithine contained as impurities needs to be less than or equal to 150 weight ppm. If the total of the contents of acetamide, 2,3,4,5-tetrahydropyridine, 2-piperidone, α-amino-ε-caprolactam, lysine, and ornithine contained as impurities in the 1,5-pentamethylene diamine exceeds 150 weight ppm, the color tone of the polyamide resin obtained through a condensation reaction between 1,5-pentamethylene diamine and dicarboxylic acid deteriorates. The total of the contents of acetamide, 2,3,4,5-tetrahydropyridine, 2-piperidone, α-amino-ε-caprolactam, lysine, and ornithine is preferably less than or equal to 100 weight ppm, more preferably less than or equal to 75 weight ppm, even more preferably less than or equal to 50 weight ppm, particularly preferably less than or equal to 25 weight ppm, and most preferably 0 weight ppm. If the total of the contents of acetamide, 2,3,4,5-tetrahydropyridine, 2-piperidone, α-amino-ε-caprolactam, lysine, and ornithine is reduced to less than or equal to 100 weight ppm, to less than or equal to 80 weight ppm, to less than or equal to 50 weight ppm, and to 0 weight ppm, the color tone of the polyamide resin obtained through the condensation reaction between the 1,5-pentamethylene diamine and a dicarboxylic acid becomes more excellent. Incidentally, in order to bring the aforementioned total amount to 0 weight ppm, there is a need to reduce the recovered amount of 1,5-pentamethylene diamine, which results in reduction of the polyamide resin color tone improvement effect relative to the cost required for purification and is economically disadvantageous. Therefore, considering the balance among the recovered amount, the cost, and the color tone improvement effect, a lower limit of the total content of the specific nitrogen-containing organic compounds mentioned above is industrially preferred to be 50 weight ppm. Incidentally, among the specific nitrogen-containing organic compounds, the content of one or more compounds can be 0 weight ppm.

Furthermore, the content of acetamide contained as an impurity in the 1,5-pentamethylene diamine is preferred to be less than or equal to 70 weight ppm. The content is more preferably less than or equal to 50 weight ppm, even more preferably less than or equal to 30 weight ppm, and most preferably 0 weight ppm. If the content of acetamide contained as an impurity in the 1,5-pentamethylene diamine is less than or equal to 70 weight ppm, the color tone of the polyamide resin obtained through the condensation reaction between the 1,5-pentamethylene diamine and a dicarboxylic acid becomes better. If the content of acetamide contained as an impurity in the 1,5-pentamethylene diamine is reduced to less than or equal to 70 weight ppm, to less than or equal to 30 weight ppm, and to 0 weight ppm, the color tone of the polyamide resin obtained through the condensation reaction between the 1,5-pentamethylene diamine and a dicarboxylic acid becomes even better.

Incidentally, in order to make the foregoing content of acetamide 0 weight ppm, there is a need to reduce the recovered amount of 1, 5-pentamethylene diamine, which results in reduction of the polyamide resin color tone improvement effect relative to the cost required for purification and is economically disadvantageous. Therefore, considering the balance among the recovered amount, the cost, and the color tone improvement effect, the lower limit of the content of acetamide is industrially preferred to be 30 weight ppm.

### (Production Method of 1,5-Pentamethylene Diamine)

The 1,5-pentamethylene diamine of the invention can be obtained by rectifying a raw material 1, 5-pentamethylene diamine. The raw material 1,5-pentamethylene diamine is preferred to be one synthesized from a biomass resource-derived compound, such as glucose or lysine, through an enzymatic reaction, a yeast reaction, a fermentation reaction, etc. These methods, compared with an organic syntheses method, can be carried out at a reaction temperature less than 100°C and therefore a side reaction is inhibited, so that a high-purity 1, 5-pentamethylene diamine in which the amount of nitrogen-containing organic compounds contained as impurities is small can be obtained. Hence, these methods are preferable. As concrete raw material 1,5-pentamethylene diamines, 1,5-pentamethylene diamines obtained by production methods described in Japanese Unexamined Patent Publication (Kokai) No. 2004-000114, Japanese Unexamined Patent Publication (Kokai) No. 2005-006650, Japanese Unexamined Patent Publication (Kokai) No. 2004-222569, and International Publication No. WO 2007/1113127 can be cited.

A lysine decarboxylase used in the enzyme method is an enzyme that converts lysine into pentamethylene diamine and is known to be present not only in Escherichia microbes represented by Escherichia coli K12 strain but in many living organisms.

As for the lysine decarboxylase preferred to be used in the invention, the lysine existing in these living organisms can be used and the lysine derived from recombinant cells in which the intracellular activity of the lysine decarboxylase has been enhanced can also be used.

As the recombinant cells, those derived from a microbe, an animal, a plant, or an insect can be preferably used. For example, in the case where an animal is used, a mouse, a rat, or cultured cells of thereof, etc. are used. In the case where a plant is used, for example, thale cress, tobacco, or cultured cells thereof are used. Furthermore, in the case where an insect is used, for example, a silkworm or cultured cells thereof are used. Furthermore, in the case where a microbe is used, for example, E. coli or the like is used.

Furthermore, a plurality of kinds of lysine decarboxylases may be combined and used.

As microbes that have such lysine decarboxylases, Bacillus halodurans (Bacillus halodurans), Bacillus subtilis (Bacillus subtilis), Escherichia coli (Escherichia coli), Selenomonas ruminantium (Selenomonas ruminantium), Vibrio cholerae (Vibrio cholerae), Vibrio parahaemolyticus (Vibrio parahaemolyticus), Streptomyces coelicolor (Streptomyces coelicolor), Streptomyces pilosus (Streptomyces pilosus), Eikenella corrodens (Eikenella corrodens), Eubacterium acidaminophilum (Eubacterium acidaminophilum), Salmonella typhimurium (Salmonella typhimurium), Hafnia alvei (Hafnia alvei), Neisseria meningitidis (Neisseria meningitidis), Thermoplasma acidophilum (Thermoplasma acidophilum), Pyrococcus abyssi (Pyrococcus abyssi), Corynebacterium glutamicum (Corynebacterium glutamicum), etc. can be cited.

The method for obtaining a lysine decarboxylase is not particularly restricted. However, for example, it is possible to culture a microbe that has a lysine decarboxylase, recombinant cells in which the intracellular activity of a lysine decarboxylase has increased, or the like in an appropriate culture medium, recover the grown cell body, and use it as a resting cell body. It is also possible to break such a cell body to prepare and use a cell-free extract liquid and use it. Further, according to need, such a cell-free extract liquid can also be purified and used.

The method of culturing a recombinant cell or a microbe that has a lysine decarboxylase in order to extract the lysine decarboxylase is not particularly restricted. However, for example, in the case where a microbe is cultured, the culture medium to be used is a culture medium that contains a carbon source, a nitrogen source, inorganic ions, and, according to need, other organic components. For example, in the case of E. coli, LB culture medium is often used. As a carbon source, sugars, such as glucose, lactose, galactose, fructose, arabinose, maltose, xylose, trehalose, or hydrolysates of ribose or starch, alcohols, such as glycerol, mannitol or sorbitol, or organic acids such as gluconic acid, fumaric acid, citric acid, or succinic acid, can be used. As the nitrogen source, inorganic ammonium salts, such as ammonium sulfate, ammonium chloride, ammonium phosphate, or ammonium phosphate, organic nitrogens such as soybean hydrolysate, ammonia gas, ammonia water, etc. can be used. Furthermore, an organic micronutrient can be contained. As the organic micronutrient, it is desirable that required substances, including various amino acids, vitamins such as vitamin B1, nucleic acids such as RNA, etc., or a yeast extract, etc. be contained in appropriate amounts. Besides these materials, inorganic ions, such as calcium phosphate, calcium sulfate, iron ion, or manganese ion are added in small amounts according to need.

The culture conditions are not particularly restricted; for example, in the case of E. coli, it is appropriate to implement the culturing for about 16 to 72 hours under aerobic conditions, and it is appropriate that the culture temperature be controlled to 30°C to 45°C and, particularly preferably, to 37°C and the culture pH be controlled to 5 to 8 and, particularly preferably, to pH 7. Incidentally, for pH adjustment, inorganic or organic acid or alkaline substances and also an ammonia gas, etc. can be used.

The grown microbe or recombinant cells can be recovered from the culture solution by centrifugal separation or the like. In order to prepare a cell-free extract liquid from the recovered microbe or recombinant cells, an ordinary method is used. That is, the microbe or recombinant cells are broken by a method such as an ultrasonication, a Dyno-mill, a French press, etc., and centrifugal separation is employed to remove a cell body residue, whereby a cell-free extract liquid is obtained.

To purify lysine decarboxylase from the cell-free extract liquid, techniques ordinarily used for purification of an enzyme, such as ammonium sulfate fractionation, ion exchange chromatography, hydrophobic chromatography, affinity chromatography, gel filtration chromatography, isoelectric point precipitation, heat treatment, pH treatment, etc., are appropriately combined and used. The purification does not necessarily need to be complete purification but is sufficient if contaminants, such as an enzyme that is other than the lysine decarboxylase and that participates in decomposition of lysine, a breakdown enzyme for a pentamethylene diamine that is the product, etc., can be removed.

The conversion from lysine to pentamethylene diamine by the lysine decarboxylase can be performed by causing the lysine decarboxylase obtained as described above to contact lysine.

With regard to the concentration of lysine in the reaction solution, there is no particular restriction. As for the amount of the lysine decarboxylase, it suffices that it is an amount that is sufficient to catalyze the reaction of converting lysine into pentamethylene diamine.

The reaction temperature is usually greater than or equal to 28°C and less than or equal to 55°C and, preferably, around 40°C. The reaction pH is usually greater than or equal to 5 and less than or equal to 8 and, preferably, about 6. As pentamethylene diamine is produced, the reaction solution turns alkaline. Therefore, in order to maintain the reaction pH, it is preferable to add an inorganic or organic acid substance. Preferably, hydrochloric acid or sulfuric acid can be used. For the reaction, either of stationary and agitation methods can be adopted. The lysine decarboxylase may be immobilized. The reaction time varies depending on conditions such as the activity of an enzyme used or the concentration of a substrate but is usually greater than or equal to 1 hour and less than or equal to 72 hours. Besides, the reaction may be continuously conducted while lysine is being supplied.

As the method for collecting the pentamethylene diamine produced as described above from the reaction liquid after the reaction ends, a method in which an ion exchange resin is used, a method in which a precipitant is used, a method in which solvent extraction is performed, a method in which simple distillation is performed, and other ordinary collection and separation methods can be adopted.

### (Reduction Method For Impurities In Raw Material 1,5-Pentamethylene Diamine)

As a method for causing the total of the contents of acetamide, 2,3,4,5-tetrahydropyridine, 2-piperidone, ε-caprolactam, α-amino-ε-caprolactam, lysine, and ornithine contained as impurities in the raw material 1,5-pentamethylene diamine to be less than or equal to 150 weight ppm, there is rectification. The rectification in the invention may be a technique that uses either of a plate column and a packed tower and may be of a continuous type or a batch type.

Furthermore, the temperature condition in the rectification is greater than or equal to 40°C and less than or equal to 200°C, preferably greater than or equal to 60°C and less than or equal to 150°C, and more preferably greater than or equal to 70°C and less than or equal to 120°C.

The pressure condition is greater than or equal to 0.2 kPa and less than or equal to 1200 kPa, preferably greater than or equal to 0.5 kPa and less than or equal to 800 kPa, and more preferably greater than or equal to 1.0 kPa and less than or equal to 500 kPa.

The theoretical plate number is greater than or equal to 5, preferably greater than or equal to 10, and more preferably greater than or equal to 20. If the theoretical plate number is less than 5, acetamide, 2,3,4,5-tetrahydropyridine, 2-piperidone, ε-caprolactam, α-amino-ε-caprolactam, lysine, and ornithine contained as impurities cannot be sufficiently removed.

Furthermore, the reflux ratio is from 1 to 100, preferably from 1 to 50, and more preferably from 1 to 10.

By performing the rectification under such conditions, it is possible to cause the total of the contents of acetamide, 2,3,4,5-tetrahydropyridine, 2-piperidone, ε-caprolactam, α-amino-ε-caprolactam, lysine, and ornithine contained as impurities in 1, 5-pentamethylene diamine to be less than or equal to 150 weight ppm.

### (Polyamide Resin)

As for the 1,5-pentamethylene diamine of the invention, the total of the contents of acetamide, 2,3,4,5-tetrahydropyridine, 2-piperidone, α-amino-ε-caprolactam, lysine, and ornithine contained as impurities is less than or equal to 150 weight ppm. Therefore, using this as a raw material of the polyamide resin makes it possible to give a polyamide resin that has good color tone.

The polyamide resin for which the 1,5-pentamethylene diamine of the invention and dicarboxylic acid are raw materials may contain a skeleton of another diamine that is other than 1,5-pentamethylene diamine, within such a range that the advantageous effects of the invention are not impaired. The polyamide is preferred to be a polyamide in which 70 mol% or more of the repeating units of the diamine component that constitute the polyamide is constituted by the 1,5-pentamethylene diamine of the invention. More preferably, 80 mol% or more thereof is so and, most preferably, 90 mol% or more thereof is so.

The another diamine skeleton may contain, for example, a structural unit derived from an aliphatic diamine such as ethylene diamine, 1,3-diaminopropane, 1,4-diaminobutane, 1,6-diaminohexane, 1,7-diaminoheptane, 1,8-diaminooctane, 1,9-diaminononane, 1,10-diaminodecane, 1,11-diaminoundecane, 1,12-diaminododecane, 1,13-diaminotridecane, 1,14-diaminotetradecane, 1,15-diaminopentadecane, 1,16-diaminohexadecane, 1,17-diaminoheptadecane, 1,18-diaminooctadecane, 1,19-diaminononadecane, 1,20-diaminoeicosane, or 2-methyl-1,5-diaminopentane, an alicyclic diamine such as cyclohexane diamine or bis- (4-aminohexyl)methane, an aromatic diamine such as xylylene diamine, etc.

Furthermore, as for the dicarboxylic acid, there is no particularly limitation but aliphatic dicarboxylic acids such as oxalic acid, malonic acid, succinic acid, glutaric acid, pimelic acid, suberic acid, azelaic acid, sebacic acid, undecanedionic acid, dodecanedioic acid, brush phosphoric acid, tetradecanedionic acid, pentadecanedionic acid, or octadecanedionic acid, aromatic dicarboxylic acids such as cyclohexane dicarboxylic acid, phthalic acid, isophthalic acid, terephthalic acid, or naphthalene dicarboxylic acid are cited. Among these, adipic acid and/or sebacic acid are suitably used.

Furthermore, the polyamide resin can contain a structural unit derived from amino acids such as 6-aminocaproic acid, 11-aminoundecanoic acid, 12-aminododecanoic acid, or para-aminomethyl benzoic acid, or lactams such as ε-caprolactam or ω-laurolactam.

It is appropriate that the relative viscosity of a polyamide resin for which the 1,5-pentamethylene diamine obtained by the invention and a dicarboxylic acid are raw materials be preferably greater than or equal to 2.0 and less than or equal to 8.0 as a relative viscosity of a 98% sulfuric acid solution with a sample concentration of 0.01 g/mL at 25°C. The relative viscosity thereof is more preferably greater than or equal to 2.05 and less than or equal to 7.0, particularly preferably greater than or equal to 2.1 and less than or equal to 6.5, and most preferably greater than or equal to 2.15 and less than or equal to 6.0. If the relative viscosity is greater than or equal to 2.0, an excellent mechanical property is obtained when the polyamide resin is made into a molded article. If the relative viscosity is less than or equal to 8.0, the melt viscosity of a composition of the polyamide resin becomes adequate and the formability thereof is excellent.

As for a polyamide component of the polyamide for which the 1,5-pentamethylene diamine obtained in the invention and a dicarboxylic acid are raw materials, a known terminal sealing agent can be added for molecular weight adjustment. As the terminal sealing agent, a monocarboxylic acid is preferred. Besides, acid anhydrides such as phthalic anhydride, monoisocyanates, monocarboxylic acid halides, monoesters, monoalcohols, etc. can be cited. As for the monocarboxylic acid that can be used as the terminal sealing agent, there is no particular restriction as long as it has reactivity with an amino group. For example, aliphatic monocarboxylic acids, such as acetic acid, propionic acid, butyric acid, valeric acid, caproic acid, caprylic acid, lauric acid, tridecylic acid, myristylic acid, palmitic acid, stearic acid, pivalic acid, or isobutylic acid, alicyclic monocarboxylic acids such as cyclohexane carboxylic acid, aromatic monocarboxylic acids, such as benzoic acid, toluic acid, α-naphthalene carboxylic acid, β-naphthalene carboxylic acid, methyl naphthalene carboxylic acid, or phenyl acetic acid, etc. can be cited. In the invention, one or more species of these monocarboxylic acids may be used.

In the invention, the polycondensation method for polyamide is not particularly limited. Generally, the polycondensation methods for polyamide include a continuous polymerization method and a batch polymerization method. With a polycondensation method for polyamide by a batch polymerization method, polypentamethylene adipamide and polypentamethylene sebacamide will be taken as examples to show an outline of the production method.

The production method for a polyamide resin for which the 1,5-pentamethylene diamine obtained by the invention and a dicarboxylic acid are raw materials is not particularly limited; for example, a heated polycondensation in which a mixture of water and a salt of a dicarboxylic acid made up of 1,5-pentamethylene diamine and adipic acid and/or sebacic acid is heated to cause a dehydration reaction to progress is generally used. Furthermore, it is possible to increase the molecular weight by solid phase polymerization after the heated polycondensation. The solid phase polymerization is caused to progress by heating in vacuum or in an inert gas in a temperature range of 100°C to the melting point thereof. In the heated polycondensation, a polyamide resin whose molecular weight is insufficient can be made to be high in molecular weight.

In the polyamide resin for which the 1,5-pentamethylene diamine obtained by the invention and a dicarboxylic acid are raw materials, other additives can be compounded according to use within such a range that the advantageous effects of the invention are not inhibited. These additives can be compounded by adding them at the time of polymerization of the polyamide or by melt-mixing them with the polyamide resin. At the time of performing the melt mixture, an extruder can be used to perform the melt mixture. Furthermore, these additives can be compounded by chip-blending master chips that contain the additives or physically mixing them with pellets of the polyamide resin and then subjecting the mixture to forming such as fiber spinning, extrusion forming, injection forming, etc.

As examples of those additives, there can be cited, for example, antioxidants and heat resistant stabilizing agents (hindered phenol-based agents, hydroquinone-based agents, phosphite-based agents, substitution products thereof, copper halide, iodine compound, etc.), weather proof agents (resorcinol-based, salicylate-based, benzotriazole-based, benzophenone-based, hindered amine-based, etc.), mold release agents and lubricants (aliphatic alcohol, aliphatic amide, aliphatic bisamide, bisurea, polyethylene wax, etc.), pigments (titanium oxide, cadmium sulfide, phthalocyanine, carbon black, etc.), dye (nigrosine, aniline black, etc.), crystal nucleus agents (talc, silica, kaolin, clay, etc.), plasticizers (p-oxy benzoic acid octyl, N-butyl benzene sulfone acid amide, etc.), antistatic agents (quaternary ammonium salt-type cation-based antistatic agents, nonion-based antistatic agents such as polyoxyethylene sorbitan monostearate, betaine-based amphoteric antistatic agents, etc.), flame retardants (melamine cyanurate, hydroxides such as magnesium hydroxide or aluminum hydroxide, polyammonium phosphate, brominated polystyrene, brominated polyphenylene oxide, brominated polycarbonate, brominated epoxy resin, or combinations of these bromine-based flame retardants and antimony trioxide, etc.), fillers (particulate, fibrous, acicular or tabular fillers or the like of graphite, barium sulfate, magnesium sulfate, calcium carbonate, magnesium carbonate, oxidation antimony, titanium oxide, aluminum oxide, zinc oxide, oxidation iron, zinc sulfide, zinc, lead, nickel, aluminum, copper, iron, stainless, glass fiber, carbon fiber, aramid fiber, bentonite, montmorillonite, synthetic mica, etc.), other polymers (other polyamides, polyethylenes, polypropylenes, polyesters, polycarbonates, polyphenylene ethers, polyphenylene sulfides, liquid crystal polymers, polysulfones, polyether sulfones, ABS resins, SAN resins, polystyrenes, etc.).

The thus obtained polyamide resin is excellent in color tone, so that even when the polyamide resin is formed into pellets, the yellowness index YI thereof can be reduced. In a preferred mode, the yellowness index YI of polypentamethylene adipamide resin pellets is less than or equal to 6.0. In a more preferred mode, it is less than or equal to 3.3. Further preferably, it is less than or equal to 2.0. A lower limit value thereof is about -15.

Incidentally, the YI value mentioned herein refers to a value measured by a method described later. The YI being low represents that the coloration is low. If the coloration is great, the range of use is limited and, furthermore, low product value may result. Therefore, the YI is preferred to be low.

### Examples

Hereinafter, the invention will be described in more detail on the basis of examples; however, the invention is not limited by these examples.

### [Analysis of Impurities Contained in 1,5-Pentamethylene Diamine]

The analysis of impurities in 1,5-pentamethylene diamine was carried out by a GC method under conditions indicated below.
GC: Shimadzu GC-2010
Column: HP-5MS, 30 m x 0.25 mm ID, 0.25µm Film
Oven: 50°C → (8°C/min) → 280°C
Inj. Temp.: 280°C
Inj. Mode: splitless
Inj. Vol.: 1.0 µL
Det. Temp: 280°C

### [Relative Viscosity (ηr) ]

0.25 g of a specimen was dissolved in 100 ml of sulfuric acid having a concentration of 98 wt% so that a sample concentration of 0.01 g/mL was reached. Using an Ostwald-type viscometer, the efflux time (T1) at 25°C was measured. Subsequently, the efflux time (T2) of the sulfuric acid whose concentration was 98 wt% alone was measured. The ratio of T1 to T2, that is, T1/T2, was defined as a relative viscosity (ηr).

### [Amount of Amino Groups]

1 g of a specimen was dissolved in 50 mL of a phenol/ethanol shuffling solution (volume ratio: phenol/ethanol = 80/20) by shaking at 30° to make a solution. This solution was subjected to neutralization titration with 0.02 N hydrochloric acid and the amount of 0.02 N hydrochloric acid required was determined. Furthermore, the phenol/ethanol mixed solvent (in the same amount as mentioned above) alone was subjected to neutralization titration with 0.02N hydrochloric acid and the amount of 0.02N hydrochloric acid required was determined. Then, from the difference therebetween, the amount of amino groups per 1 g of the specimen was determined.

### [Yellowness index (YI)]

Using a color computer made by Suga Test Instruments Co., Ltd., the YI value of pellets was measured. As for the measurement method, measurement was performed according to JIS standard K7373 (2006) (an optical characteristic test method for plastics).

### Reference example 1 (culture of a lysine decarboxylase-expressing strain)

The plasmid pcadA220 that highly expresses the lysine decarboxylase (CadA) was constructed by a method described in Japanese Unexamined Patent Publication (Kokai) No. 2008-193898. The pcad A220 is a plasmid that has an E. coli-derived lysine decarboxylase gene (CadA) linked downstream of the construction expression-type promoter of a modified type of the acid phosphatase gene of the Enterobacter genus and the ribosome binding site thereof and that constructively highly expresses the CadA. E. coli strain JM10 (made by Takara Shuzo Co., Ltd.) was transformed with this plasmid. The obtained transformed strain was named E. coli cadA220. This bacterial strain was pre-cultured in LB culture medium. Then, 26 ml of the culture solution was inoculated to 26 L of a seed culture medium (25 g/L Glucose, 1 g/L MgSO₄·7H₂O, 2 g/L KH₂PO₄, 5 g/L (NH₄)₂SO₄, soybean hydrochloric acid hydrolysate equivalent to 0.45 g/L inorganic nitrogen, 20 mg/L FeSO₄·7H₂O, 20 mg/L MnSO₄·5H₂O, Thiamine HCl, 0.1 ml/L of an antifoaming agent, pH 5.0), and aerated and agitated culture was performed at 28°C, 250 rpm, pH 7, and an aeration amount of 26 L/min. The culture pH was adjusted with ammonia. After 22 hours, 1. 3 L of the seed culture solution was inoculated to 26 L of a main culture medium (whose culture medium composition was the same as that of the seed culture medium) and aerated and agitated culture was performed for 12 hours at an aeration amount of 26 L/min, 30°C, 250 rpm, pH 7, and an aeration amount of 26 L/min. The culture medium pH was adjusted with ammonia. After the sugars in the main culture medium were consumed, the culture was terminated to obtain an E. coli cadA220 culture solution.

Before being subjected to reaction, this E. coli cadA220 culture solution was adjusted in pH to 6.5 with sulfuric acid and then a heating treatment was carried out for 1 hour at 55°C. The activity following the heating was measured and found to be 730 U/ml. Incidentally, the measurement of the lysine decarboxylase activity was performed according to an established method (Soda Kenji, and Misono Haruo, Lectures on Biochemical Experiments, vol. 11, Book One, pp. 179-191 (1976)).

### Reference example 2 (production of 1,5-pentamethylene diamine)

As a raw material lysine, LLB 50 (a 50%(W/V) lysine base solution, AJINOMOTO EUROLYSINE S.A.S.) was used. 650 L of a reaction liquid was prepared so as to achieve final concentrations of 200 g/L for lysine base, 26.5 mg/L for pyridoxal phosphoric acid (made by Wako Pure Chemical Industries, Ltd.), and 380 U/ml for lysine decarboxylase (prepared in Reference example 1). Incidentally, the reaction liquid was adjusted in pH to 7 with sulfuric acid and an enzyme solution was added to start the reaction. While the pH was kept at 7 with sulfuric acid, the reaction was conducted at 37°C for 10 hours. After it was confirmed that lysine was completely consumed, the reaction was terminated. After the reaction ended, the pH of the reaction liquid was lowered to 5 with sulfuric acid and heating was performed at 80°C for 30 minutes to kill bacteria. Thus, a reaction liquid containing 1,5-pentamethylene diamine-sulfuric acid salt was prepared. To this solution, activated carbon was added in an amount of 10 wt% relative to the 1,5-pentamethylene diamine. After 1 hour of stirring, activated carbon and cell body were separated from the 1,5-pentamethylene diamine solution by using a filter press separator. The 1,5-pentamethylene diamine-sulfuric acid salt and at least two-time mol of calcium hydroxide were added to the obtained filtrate to convert the 1,5-pentamethylene diamine-sulfuric acid salt into 1, 5-pentamethylene diamine, and calcium sulfate produced as precipitate was separated by centrifugation. The obtained supernatant was concentrated to about 45 wt% and then subjected to distillation to obtain 1,5-pentamethylene diamine. The contents of impurities in the obtained 1,5-pentamethylene diamine were as shown in Table 1.

### Example 1

1 L of the 1, 5-pentamethylene diamine obtained in Reference example 2 was placed in a bottom of a distillation column (theoretical plate number being 27) filled with Heli-Pak No. 4, and distillation was carried out at an internal temperature of 105°C (heater temperature of 150°C) at a pressure of 1.8 kPa and a reflux ratio of 2. The contents of nitrogen-containing organic compounds in the purified 1,5-pentamethylene diamine obtained were as shown in Table 1.

While an aqueous solution obtained by dissolving 576.4 g of the purified 1,5-pentamethylene diamine in 1400 g of ion exchanged water was being stirred in an ice bath, 823.6 g of adipic acid (made by Tokyo Chemical Industry Co., Ltd.) was added little by little. In the vicinity of the neutralization point, the solution was warmed in a 40°C-water bath so that the internal temperature was 33°C. Thus, 2800 g of a 50-weight-% aqueous solution of 1,5-pentamethylene diamine-adipic acid salt at pH 8.32.

The obtained 50-wt% aqueous solution of 1,5-pentamethylene diamine-adipic acid salt was placed in a batch type polymerization vessel having an internal volume of 5 L and equipped with a heat medium jacket and an agitator having a spiral-band stirring vane (raw material preparation step).

Next, the inside of the polymerization vessel was tightly closed, and nitrogen substitution was sufficiently performed, and then the heat medium was heated to concentrate the aqueous solution (concentration step). At this time, while the in-vessel pressure (gauge pressure) was controlled at 0.2 MPa with the in-vessel temperature at 200°C, concentration was performed so that the concentration of the raw material in the aqueous solution became 85 wt%. The concentration in the aqueous solution inside the vessel was judged from the amount of distillate water.

Then, after the concentration was terminated, the heat medium temperature was raised to 290°C so that in-vessel pressure (gauge pressure) reached 1.7 MPa (pressure raising step). After that, the in-vessel pressure (gauge pressure) was controlled at 1.7 MPa and kept until the in-vessel temperature became 255°C (pressure controlling step). Furthermore, the heat medium temperature was changed to 285°C, and pressure was discharged to the atmospheric pressure over 50 minutes (pressure discharging step). Furthermore, the in-vessel pressure (gauge pressure) was reduced to -13 kPa and was kept thereat for 30 minutes to stop the polymerization reaction (pressure reduction step). After that, a nitrogen pressure of 0.5 MPa(absolute pressure) was applied in the vessel to extrude the polyamide resin obtained by the polymerization into a shape of a strand having a diameter of about 3 mm, which was cut into a length of about 4 mm to obtain pellets (ejection step).

The relative viscosity of the obtained polyamide resin was 2.70, the amino end group amount thereof was 5.63 x 10⁻⁵ mol/g, and the YI was 1.6.

### Example 2

1 L of the 1, 5-pentamethylene diamine obtained in Reference example 2 was placed in the bottom of a distillation column (theoretical plate number being 15) filled with Heli-Pak No. 4, and distillation was carried out at an internal temperature of 105°C (heater temperature of 150°C) at a pressure of 1.8 kPa and a reflux ratio of 2. The contents of impurities in the purified 1,5-pentamethylene diamine obtained were as shown in Table 1.

Pellets of polyamide resin were obtained in substantially the same manner as in Example 1, except that the foregoing purified 1,5-pentamethylene diamine was used.

The relative viscosity of the obtained polyamide resin was 2.71, the amino end group amount thereof was 5.65 x 10⁻⁵ mol/g, and the YI was 3.3.

### Comparative example 1

700 g of the 1,5-pentamethylene diamine obtained in Reference example 2 was placed in a 1-L three-necked flask, and simple distillation was carried out at 80°C and 1.0 kPa. The contents of impurities in the purified 1,5-pentamethylene diamine obtained were as shown in Table 1.

Pellets of a polyamide resin composition were produced by substantially the same method as in Example 1, except that the foregoing purified 1,5-pentamethylene diamine was used. The obtained polyamide resin composition was slightly colored light yellow, the relative viscosity thereof was 2.65, the amino terminal amount was 6.79 x 10⁻⁵ mol/g, and the YI was 6.3.

### Comparative example 2

Pellets of a polyamide resin composition were produced by substantially the same method as in Example 1, except that the 1,5-pentamethylene diamine produced in Reference example 1 was used. The obtained polyamide resin composition was slightly colored light yellow, the relative viscosity thereof was 2.67, the amino terminal amount was 5.62 x 10⁻⁵ mol/g, and the YI was 11.6.

**[Table 1]**

| | | | | Unit: weight ppm | |
|---|---|---|---|---|---|
| | | Example 1 | Example 2 | Comparative example 1 | Reference example 2 |
| | Acetamide | 20 | 47 | 79 | 131 |
| | 2,3,4,5-Tetrahydropyridine | n.d. | 11 | 3 | 13 |
| | 2-Piperidone | n.d. | n.d. | 16 | 26 |
| Impurity | α-Amino-ε-caprolactam | n.d. | n.d. | 47 | 61 |
| content | Lysine | n.d. | n.d. | 2 | 239 |
| | Ornithine | n.d. | n.d. | 17 | 54 |
| | Arginine | n.d. | n.d. | n.d. | n.d. |
| | Total impurity content | 20 | 58 | 164 | 524 |

### Industrial Applicability

According to the invention, it becomes possible to obtain a 1,5-pentamethylene diamine that gives a polyamide resin composition having a good color tone by causing the total of the contents of acetamide, 2,3,4,5-tetrahydropyridine, 2-piperidone, ε-caprolactam and α-amino-ε-caprolactam, lysine, and ornithine that the 1,5-pentamethylene diamine contains as impurities to be less than or equal to 150 weight ppm.

## Claims

1. A 1,5-pentamethylene diamine **characterized in that** a total content of acetamide, 2,3,4,5-tetrahydropyridine, 2-piperidone, α-amino-ε-caprolactam, lysine, and ornithine contained as impurities is less than or equal to 150 weight ppm.

2. The 1,5-pentamethylene diamine according to Claim 1, **characterized in that** a content of the acetamide is less than or equal to 70 weight ppm.

3. The 1,5-pentamethylene diamine according to any of Claims 1 to 2, which is produced by a biomass resource-derived lysine.

4. A production method for the 1,5-pentamethylene diamine according to any one of Claims 1 to 3, **characterized in that** the 1,5-pentamethylene diamine is rectified at a temperature greater than or equal to 40°C and less than or equal to 200°C and a pressure greater than or equal to 0.2 kPa and less than or equal to 1200 kPa and with a theoretical plate number greater than or equal to 5.
